# EUROPÄISCHE PATENTANMELDUNG

(11) **EP 1 674 829 A2**
(43) Veröffentlichungstag der Anmeldung: **28.06.2006**
(21) Anmeldenummer: 05028089.0
(22) Anmeldetag: 21.12.2005
(51) Int. Cl.: G01D 3/10

(54) **System zum Anschluss mobiler Messgeräte an Datenerfassungseinrichtungen**

(30) Priorität: 21.12.2004 DE 102004061541
(71) Anmelder: Actimon GmbH & Co. KG, 69190 Walldorf (DE)
(72) Erfinder: Wegertseder, Dominik, 85540 Haar (DE); Schweizer, Thomas, 85560 Ebersberg (DE)
(74) Vertreter: Müller-Boré & Partner Patentanwälte

(57) **Zusammenfassung**

System zum Rückgewinnen von dargestellten Informationen aus Displayansteuerungssignalen und Weiterleitung an zentrale oder dezentrale Datenverarbeitungseinrichtungen vorgesehen, umfassend
- zumindest eine Schnittstelle, die ausgelegt ist, auf mindestens zwei Signalleitungen eines Displays, welches ausgelegt ist Displaydaten darzustellen, mittelbar oder unmittelbar zuzugreifen,
- zumindest eine Signalwandlereinheit, welche mit der zumindest einen Schnittstelle in Signalverbindung steht bzw. bringbar ist, um Ansteuersignale des Displays über die Schnittstelle zu erfassen und in Wandlersignale umzusetzen,
- zumindest eine Auswerteeinheit, die mit der Signalwandlereinheit in Signalverbindung steht und Mittel aufweist, um aus den Wandlersignalen die Displaydaten zurückzugewinnen und über mindestens eine Signalverbindung die zurückgewonnenen Displaydaten auszugeben.

## Beschreibung

Die vorliegende Erfindung betrifft ein mobiles Messgerät, das Messergebnisse auf einem Display anzeigt, wobei Mittel zur Erfassung und Weitergabe der Messwerte an eine elektronische Datenerfassungseinrichtung zur weiteren Bearbeitung vorgesehen sind.

Mobile Messgeräte zur Erfassung von Daten sind aus dem Stand der Technik bekannt. So ist beispielsweise ein Handvoltmeter bekannt, mit welchem eine Spannung gemessen werden kann und die Spannung auf einem Display angezeigt wird. Im medizinischen Umfeld sind Blutdruckmesser bekannt, welche an einem Handgelenk einer Person getragen werden und über ein Display entsprechende Daten ausgeben. Alle Messgeräte weisen bestimmte Ein- und/oder Ausgabeelemente zur Mensch-Maschine Kommunikation auf. Dies sind z.B. Schalter, Taster zur Eingabe von Informationen, sowie z.B. Signalleuchten und/oder Displays (z.B. LCD, LED, Elektroluminiszenz) und/oder akustische Signalwandler (z.B. elektromagnetische oder Piezo-Lautspecher) zur Ausgabe von Informationen an den Anwender. Weiterhin sind Messgeräte mit Schnittstellen zu elektronischen Datenverarbeitungseinrichtungen bekannt, aber nur wenige Messgeräte sind mit einer solchen Schnittstelle erhältlich und der Preis von Geräten mit Schnittstellen liegt erheblich über dem von Messgeräten ohne Schnittstellen.
In modernen Messsystemen und Messnetzen ist es aus Kosten-, Dokumentations-und Zuverlässigkeitsanforderungen nötig, alle erfassten Messungen beliebiger Parameter automatisch und elektronisch an die nachfolgende elektronische Datenverarbeitung weiterzuleiten. Teilweise ist es zusätzlich nötig, Messungen automatisch anzustoßen und/oder Bedienungen zu erkennen und zu dokumentieren. Zum Beispiel werden zur Zeit Messung physiologischer Parameter in Krankenhäusern fast ausschließlich von den Krankenschwestern durchgeführt und anschließend die Messergebnisse von Hand in das Krankenhaussystem zur elektronischen Datenverarbeitung eingegeben.

Es ist Aufgabe der vorliegenden Erfindung, jedes beliebige mobile mit einem Display und/oder Audiosiganlgeber ausgestattete Messgerät zur Erfassung von beliebigen Parametern, bevorzugterweise physiologischer Parametern eines Lebewesens, kostengünstig mit einer Schnittstelle aus- oder nachzurüsten.

Diese Aufgabe wird durch ein System zum Rückgewinnen von dargestellten Informationen aus Displayansteuerungssignalen und Weiterleitung an zentrale oder dezentrale Datenverarbeitungseinrichtungen gemäß Anspruch 1 sowie ein System zur Steuerung und Erfassen von Messwerten eines mobilen Messgerätes gemäß Anspruch 22 gelöst. Bevorzugte Ausführungsformen sind Gegenstand der abhängigen Unteransprüche.

Die Erfindung nutzt vorzugsweise den Effekt, dass Displays, die eine größere Anzahl einzeln ansteuerbarer Segmente aufweisen, in einer Matrix angeordnet werden und im so genannten zeitmultiplex Verfahren angesteuert werden. Dies bedeutet, dass die Segmente zu Gruppen zusammengefasst werden. Innerhalb der Gruppen wird eine Ansteuersignalleitung der Segmente zusammengefasst. Jedes Segment ist in zwei orthogonale Gruppen eingeordnet. Diese Ansammlung der jeweilig orthogonalen Gruppen werden oft als Zeilen und Spalten bezeichnet. Die Selektion eines Segmentes erfolgt durch Aktivierung einer Zeile und einer Spalte in der Matrix. Ferner nutzt die Erfindung vorzugsweise den Effekt, dass zur Vermeidung von Einbrenneffekten und Degradation der Displays, Verbesserung des Kontrasts, sowie der Vermeidung von Störung von Nachbarelementen sehr spezielle elektrische Signalverläufe im Bezug auf Spannungs- und Zeitverlauf an die Ansteuerleitungen des Displays angelegt werden müssen. So können Messgeräte durch Datenverarbeitungseinheiten gesteuert werden, indem die Bedienelemente (z.B. Schalter und Taster) durch Steuerelemente ersetzt oder parallelgeschaltet werden. Steuerelemente können Relais, Bipolar- oder Feldeffekttransistoren sein.

Erfindungsgemäß ist ein System zum Rückgewinnen von dargestellten Informationen aus Displayansteuerungssignalen und Weiterleitung an zentrale oder dezentrale Datenverarbeitungseinrichtung vorgesehen, umfassend
- zumindest eine Schnittstelle, die auf mindestens zwei Signalleitungen des Displays zugreift,
- zumindest eine Signalwandlereinheit, welche mit dem Display in Signalverbindung steht, um die meist analogen Displayansteuersignale in Digitale Signale umzusetzen,
- Zumindest eine Auswerteeinheit, die mit der Signalwandeleinheit in Signalverbindung steht und aus deren übertragener Information die Messwerte zurückgewinnt und über mindestens eine Signalverbindung die zurückgewonnenen Messdaten, Daten in digitaler Form an eine Datenverarbeitungseinrichtung weitergibt.
   In anderen Worten ist erfindungsgemäß ein System zum Rückgewinnen von dargestellten Informationen aus Displayansteuerungssignalen und zur Weiterleitung an zentrale oder dezentrale Datenverarbeitungseinrichtungen vorgesehen, umfassend
- zumindest eine Schnittstelle, die ausgelegt ist, auf mindestens zwei Signalleitungen eines Displayszur Darstellung von Displaydaten, mittelbar oder unmittelbar zuzugreifen,
- zumindest eine Signalwandlereinheit, welche mit der zumindest einen Schnittstelle in Signalverbindung steht bzw. bringbar ist, um Ansteuersignale des Displays über die Schnittstelle zu erfassen und in Wandlersignale umzusetzen,
- zumindest eine Auswerteeinheit, die mit der Signalwandlereinheit in Signalverbindung steht und Mittel aufweist, um aus den Wandlersignalen die Displaydaten zurückzugewinnen und über mindestens eine Signalverbindung die zurückgewonnenen Displaydaten auszugeben.

Der Zugriff auf die Signalleitungen des Displays mittels der Schnittstelle kann vorteilhafterweise unmittelbar erfolgen, indem ein elektrisches Signal von den Signalleitungen abgegriffen wird, beispielsweise durch eine Verdrahtung. Alternativ kann der Zugriff auf die Signalleitungen des Displays mittels der Schnittstelle vorteilhafterweise mittelbar erfolgen, beispielsweise durch induktive Signalübertragung von den Signalleitungen an die Schnittstelle. Ein direkter körperlicher Kontakt der Schnittstelle mit den Signalleitungen ist somit nicht notwendig. Die Signalwandlereinheit ist vorteilhafterweise ausgelegt, Ansteuersignale des Displays über die Schnittstelle zu erfassen und in Wandlersignale umzusetzen, wobei die Umsetzung vorzugsweise in digitale Wandlersignale erfolgt. Die Auswerteeinheit ist vorteilhafterweise ausgelegt, aus den Wandlersignalen die Displaydaten zurückzugewinnen. Die Displaydaten umfassen zweckmäßigerweise die Meßwerte, welche auf dem Display dargestellt werden. Diese Meßwerte können bei einem Meßgerät zur Messung von physiologischen Parametern z. B. Temperatur, Blutdruck, Herzfrequenz, EKG, Gewicht, Bewegung (Geschwindigkeit und Beschleunigung) sein. Es wird somit vorteilhafterweise ein System bereitgestellt, das fähig ist, in einer einfachen Art und Weise von einer beliebigen Vorrichtung, welche Information auf einem Display ausgibt, die Information zu erfassen und weiterzuverarbeiten oder beliebig weiter zu nutzen. Hierbei ist eine substantielle Veränderung der Vorrichtung nicht notwendig, da die zur Erfassung der Information und Weiterverarbeitung notwendigen Einrichtungen und Logik in dem erfindungsgemäßen System vorgesehen sind.

Vorteilhafterweise ist es möglich das System modular auszubilden, um an standardisierte bzw. vorbestimmte oder vorbestimmbare Messgeräte anordenbar zu sein. Infolgedessen wird vorteilhafterweise ein System geschaffen, das zum Rückgewinnen von dargestellten Informationen aus Displayansteuerungssignalen und Weiterleitung an zentrale oder dezentrale Datenverarbeitungseinrichtung ausgebildet ist und an bestehende Messgeräte nachrüstbar ist.Hierbei ist das System zum Rückgewinnen von dargestellten Informationen aus Displayansteuerungssignalen und zur Weiterleitung an zentrale oder dezentrale Datenverarbeitungseinrichtung vorteilhafterweise ausgebildet, Daten in mobilen physiologischen Messgeräten zu erfassen. Hierzu können insbesondere der Blutdruck, der Puls, die Temperatur, die Atmungsfrequenz, das EKG (d.h. die Stromspannungsschwankung im Körper bzw. im/am Herzen) und der Sauerstoffsättigungsgehalt des Blutes zählen.

Vorteilhafterweise ist das System mobil ausgebildet und vorzugsweise ausgebildet ist, Daten in mobilen physiologischen Messgeräten zu erfassen. Das System zum Rückgewinnen von dargestellten Informationen aus Displayansteuerungssignalen und Weiterleitung an zentrale oder dezentrale Datenverarbeitungseinrichtung ist vorteilhafterweise mobil ausgebildet und am Messobjekt angeordnet bzw. anordenbar. Vorzugsweise kann diese auch in unmittelbarer räumlicher Nähe zum Messobjekt angeordnet sein, wobei unter "unmittelbarer räumlicher Nähe" vorzugsweise ein Bereich bis zu 10 m, besonders bevorzugterweise ca. 1 m zu verstehen ist. Unter "mobil" ist vorzugsweise ein Zustand zu verstehen, in welchem das erfindungsgemäße System bzw. die Datenverarbeitungseinrichtung sowohl hinsichtlich ihrer räumlichen Abmessungen als auch hinsichtlich ihres Gewichts durch eine Person bzw. ein Lebewesen bewegbar ist. Vorzugsweise ist das mobile Messgerät leichter als ca. 1 kg. Die räumlichen Abmessungen bzw. die räumliche Ausdehnung der Datenerfassungseinrichtung ist vorteilhafterweise geringer als ca. 10 dm³ und besonders bevorzugterweise geringer als ca. 1 dm³.

Vorzugsweise ist die Signaiwandiereinheit ausgelegt, den Zeit- und Spannungsverlauf der Ansteuersignale des Displays in digitale Signalverläufe umzuwandeln und an die Auswerteeinheit weiterzuleiten. Die Signalwandlereinheit ist somit vorteilhafterweise ausgelegt, den Zeit- und Spannungsverlauf der Displayansteuersignale in digitale Signalverläufe umzusetzen und an die Auswerteeinheit weiterzuleiten.

Bevorzugterweise ist die Signalwandlereinheit als Analog-nach-Digital-Wandler der Signale ausgelegt. In anderen Worten erfolgt diese Umsetzung durch Analog nach Digitalwandlung der Signale. Zum Beispiel mit Analog-Digitalwandlern nach dem Prinzip der sukzessiven Approximation.

Vorteilhafterweise ist die Signalwandlereinheit ausgelegt, eine Klassifizierung der Wandlersignale in eine vorbestimmte oder vorbestimmbare Anzahl von vorzugsweise vorbestimmten oder vorbestimmbaren Zuständen vorzunehmen. Hierbei wird bei der Implementierung der Wandler auf die Signaleigenheiten des Displays angepasst. Im bevorzugten Fall ist die Signalwandlereinheit ausgelegt, eine Klassifizierung der Wandlersignale in zwei vorzugsweise vorbestimmte oder vorbestimmbare Zustände vorzunehmen. Bei einem LCD Display bedeutet dies vorteilhafterweise eine Spannungsklassifizierung in nur 2 Zustände, die durch Komperatoranordnungen detektiert werden kann. In anderen Worten wird bei der Implementierung des bzw. der Wandler entsprechend der Displayansteuersignale angepaßt.

Bevorzugterweise ist die Signalwandlereinheit ausgelegt, von den Displayansteuerungssignalen, welche zur Darstellung von Information auf dem Display mittels eines matrixartigen Bildaufbaus ausgelegt sind, die Spannungsmaxima der den Zeilen zugeordneten Displayansteuerungssignalen und die Spannungsminima der den Spalten zugeordneten Displayansteuerungssignalen zu detektieren. Zusätzlich oder alternativ kann die Signalwandlereinheit ausgelegt sein, von den Displayansteuerungssignalen, welche zur Darstellung von Information auf dem Display mittels eines matrixartigen Bildaufbaus ausgelegt sind, die Spannungsmaxima der den Spalten zugeordneten Displayansteuerungssignalen und die Spannungsminima der den Zeilen zugeordneten Displayansteuerungssignalen zu detektieren. In anderen Worten werden nur die Spannungsmaxima der Zeilen und die Spannungsminima der Spalten und/oder auch nur die Spannungsmaxima der Spalten und die Spannungsminima der Zeilen detektiert.

Vorteilhafterweise ist die Signalwandlereinheit ausgelegt, von den Displayansteuerungssignalen, welche zur Darstellung von Information auf dem Display mittels eines matrixartigen Bildaufbaus ausgelegt sind, alle Spannungsminima und -maxima der den Spalten und Zeilen zugeordneten Displayansteuerungssignale zu detektieren. Insbesondere können charakteristische Merkmale der Displayansteuersignale detektiert werden.

Vorteilhafterweise weist die Signalwandlereinheit Zeilen- und/oder Spaltenwandler auf, deren Anzahl vorzugsweise geringer als die Anzahl der Zeilen bzw. Spalten ist, welche den Displayansteuerungssignalen zur Darstellung von Information auf dem Display mittels eines matrixartigen Bildaufbaus entsprechen. Die Ansteuerung der Zeilen oder Spalten erfolgt vorteilhafterweise zeitlich deterministisch und die Information der restlichen Zeilen bzw. Spalten in der Auswerteeinheit kann dadurch zurückgewonnen werden.

Bevorzugterweise weist die Signalwandlereinheit einen einzigen Zeilen- und/oder Spaltenwandler auf. Die Ansteuerung der Zeilen oder Spalten erfolgt vorteilhafterweise zeitlich deterministisch und die Information der restlichen Zeilen bzw. Spalten in der Auswerteeinheit kann dadurch zurückgewonnen werden. Somit können die Anzahl der Zeilen- oder Spaltenkomperatoren auf eins reduziert werden, da die Ansteuerung der Zeilen oder Spalten zeitlich deterministisch erfolgt und dadurch die Informationen über die restlichen Zeilen bzw. Spalten in der Auswerteeinheit zurückgewonnen werden können.

Vorteilhafterweise weist die Signalwandlereinheit Mittel zur adaptiven Rückgewinnung der nicht gewandelten Spaltensignale auf. In anderen Worten kann die Rückgewinnung der restlichen Spaltensignale adaptiv erfolgen. Dies bedeutet, das durch die information der Anzahl der Spalten und die Messung der Zeit zwischen zwei Aktivierungen einer Spalte die zeitliche Abfolge der Spaltenansteuerung abgeleitet werden kann.

Vorzugsweise ist die Auswerteeinheit ausgelegt, die zeitliche Abfolge der Signale der Signalwandlereinheit zu erfassen und die Displaydaten mittels der Wandlersignale, in vorzugsweise digitale Informationen des Messwerts, umzuwandeln.

Bevorzugterweise ist die Auswerteeinheit ausgelegt, im Zeitmultiplex eintreffende Daten zu erfassen und zumindest temporär zu speichern, bis ein komplettes Abbild der Displaydaten vorliegt, und diese dann elektronisch zu analysieren. Somit werden vorteilhafterweise die im Zeitmultiplex eintreffenden Daten gesammelt, bis ein komplettes Abbild der Displaydaten vorliegt und diese dann elektronisch analysiert.

Bevorzugterweise ist die Auswerteeinheit als festverdrahtete Logik oder als Mikroprozessorsystem mit Software ausgebildet.

Vorteilhafterweise weist die Auswerteeinheit Übertragungsmittel auf, um die rückgewonnen Displaydaten bzw. Messdaten weiterzuleiten. Somit können die rückgewonnen Messdaten weitergeleitet werden oder Veränderungen von Displayinhalten vorgenommen werden.

Weiterhin vorteilhafterweise weist die Auswerteeinheit Komparatormittel auf, um eine Veränderung des Displayinhalts zu erfassen und diese weiterzuleiten, wobei die Komparatormittel ausgelegt sind, anhand der Veränderung des Displayinhaltes Zustandsänderungen der Displaydaten zu erkennen. Die Veränderung des Displayinhaltes kann als Erkennung dienen, dass das das Messgerät eingeschaltet wurde, einen Fehlerzustand anzeigt, oder nur Daten vorliegen.

Bevorzugterweise werden die durch die Auswerteeinheit rückgewonnenen Daten drahtgebunden oder drahtlos (z.B.Funk) mittels vorzugsweise drahtloser Übertragungsmittel übertragen.

Das beschriebene System zum Rückgewinnen von dargestellten Informationen aus Displayansteuerungssignalen und Weiterleitung an zentrale oder dezentrale Datenverarbeitungseinrichtung kann wahlweise aus diskreten Komponenten aufgebaut werden oder ganz oder teilweise in einen integrierten Schaltkreis integriert werden.

Bevorzugterweise sind Mittel vorgesehen, um vor der Weiterleitung der Daten an die zentrale oder dezentrale Datenverarbeitungseinrichtung eine Datenvorverarbeitung und/oder Speicherung der Daten auszuführen. Das beschriebene System zum Rückgewinnen von dargestellten Informationen aus Displayansteuerungssignalen und Weiterleitung an zentrale oder dezentrale Datenverarbeitungseinrichtung kann wahlweise vor der Weiterleitung der Daten an die zentrale oder dezentrale Datenverarbeitungseinrichtung eine Datenvorverarbeitung und/oder Speicherung der Daten vornehmen. Vorteilhafterweise ermöglicht dies z.B. eine Filterung der Daten und/oder ein Zwischenspeichern der Daten z.B. beim Fehlen der Verbindung zur Datenverarbeitungseinrichtung, bis eine bestimmte Anzahl von Messwerten gesammelt wurden oder die Daten durch die Datenverarbeitungseinrichtung abgerufen werden.

Das oben beschriebene System zum Rückgewinnen von dargestellten Informationen aus Displayansteuerungssignalen und Weiterleitung an zentrale oder dezentrale Datenverarbeitungseinrichtung kann in analoger Weise alternativ oder zusätzlich zum Erfassen von Ansteuersignalen eines Audioschallwandler zur Rückgewinnung der an den Menschen übertragenen Information ausgelegt sein, wobei das System aufweist
- zumindest eine Schnittstelle, die ausgelegt ist, auf mindestens eine Signalleitung des Audioschallwandlers mittelbar oderunmittelbar zuzugreifen,
- zumindest eine Signalwandlereinheit, welche mit der Schnittstelle in Signalverbindung steht, um die Audioschallwandleransteuersignale in vorzugsweise digitale Signale umzusetzen,
- zumindest eine Auswerteeinheit, die mit der Signalwandeleinheit in Signalverbindung steht und Mittel aufweist, um aus den Signalen bzw. deren übertragener Information die Audioinformation zurückzugewinnen und über mindestens eine Signalverbindung die zurückgewonnenen Signale bzw. Audioinformation in vorzugsweise digitaler Form an eine Datenverarbeitungseinrichtung weiterzugeben.

Vorteilhafterweise sind diese Audioinformationen Signaltonabgaben, Tonhöhen, Tonfolgen, Lautstärken, Tonlängen.

Vorteilhafterweise ist es möglich das System zum Erfassen der Ansteuersignale eines Audioschallwandler zur Rückgewinnung der an den Menschen übertragenen Information an bestehenden Messgeräten nachrüstbar auszuführen.

Das System zum Erfassen der Ansteuersignale einesAudioschallwandler zur Rückgewinnung der an den Menschen übertragenen Information ist vorteilhafterweise ausgebildet Daten in mobilen physiologien Messgeräten zu erfassen. Hierzu können insbesondere der Blutdruck, der Puls, die Temperatur, die Atmungsfrequenz, das EKG (d.h. die Stromspannungsschwankung im Körper bzw. im/am Herzen) und der Sauerstoffsättigungsgehalt des Blutes zählen.

Das System zum Erfassen der Ansteuersignale einesAudioschallwandler zur Rückgewinnung der an den Menschen übertragenen Information ist vorteilhafterweise mobil ausgebildet und am Messobjekt angeordnet. Vorzugsweise kann diese auch in unmittelbarer räumlicher Nähe Messobjekt angeordnet sein, wobei unter "unmittelbarer räumlicher Nähe" vorzugsweise ein Bereich bis zu 10 m, besonders bevorzugterweise ca. 1 m. Unter "mobil" ist vorzugsweise ein Zustand zu verstehen, in welchem die Datenverarbeitungseinrichtung sowohl hinsichtlich ihrer räumlichen Abmessungen als auch hinsichtlich ihres Gewichts durch eine Person bzw. ein Lebewesen bewegbar ist. Vorzugsweise ist das mobile Messgerät leichter als ca. 1 kg. Die räumlichen Abmessungen bzw. die räumliche Ausdehnung der Datenerfassungseinrichtung ist vorteilhafterweise geringer als ca. 10 dm³ und besonders bevorzugterweise geringer als ca. 1 dm³.

Die Signalwandlereinheit ist ausgelegt, den Zeit- und Spannungsverlauf der Audioansteuersignale in digitale Signalverläufe umzusetzen und an die Auswerteeinheit weiterzuleiten.
Bevorzugterweise geschieht diese Umsetzung durch Analog nach Digitalwandlung der Signale. Zum Beispiel mit Analog-Digitalwandlern nach dem Prinzip der sukzessiven Approximation.
Vorteilhafterweise wird bei der Implementierung der Wandler auf die Signaleigenheiten des Schallwandlers angepasst. Im bevorzugten Fall eines Piezo Schallwanders bedeutet dies eine Spannungsklassifizierung in 2 Zustände, die durch Komperatoranordnungen detektiert werden kann. Es werden erfindungsgemäß nur die Spannungsmaxima detektiert. Wahlweise auch nur die Spannungsmaxima detektiert. Wahlweise werden alle Spannungsminima und -maxima detektiert.

Die Auswerteeinheit ist ausgelegt die zeitliche Abfolge der Signale der Signalwandlereinheit zu erfassen und in digitale Informationen des Messwerts umzuwandeln.

Bevorzugterweise ist die Auswerteeinheit als festverdrahtete Logikschaltung oder als Mikroprozessorsystem mit Software ausgebildet.

Bevorzugterweise werden die durch die Auswerteeinheit rückgewonnenen Daten drahtgebunden oder drahtlos (z.B. Funk) übertragen.

Das System zum Erfassen der Ansteuersignale einesAudioschallwandler zur Rückgewinnung der an den Menschen übertragenen Information kann wahlweise vor der Weiterleitung der Daten an die zentrale oder dezentrale Datenverarbeitungseinrichtung eine Datenvorverarbeitung und/oder Speicherung der Daten vornehmen. Vorteilhafterweise ermöglicht dies z.B. eine Filterung der Daten und/oder ein Zwischenspeichern der Daten z.B. beim Fehlen der Verbindung zur Datenverarbeitungseinrichtung, bis eine bestimmte Anzahl von Daten gesammelt wurden oder die Daten durch die Datenverarbeitungseinrichtung abgerufen werden.

Weiterhin erfindungsgemäß ist ein System zur Steuerung und Erfassen von Messwerten eines mobilen Messgerätes, umfassend
- zumindest ein erfindungsgemäßes System zum Rückgewinnen von dargestellten Informationen aus Displayansteuerungssignalen und Weiterleitung an zentrale oder dezentrale Datenverarbeitungseinrichtung,
- zumindest eine Anordnung zur Steuerung von Messgeräte durch zentrale oder dezentrale Datenverarbeitungseinheiten,
- zumindest eine Schnittstelle zu einer elektronischen Datenverarbeitungseinrichtung

Vorteilhafterweise erlaubt das System zur Steuerung und Erfassen von Messwerten eines mobilen Messgerätes ein durch die Datenerfassungseinrichtung angestoßenes Durchführen von Messungen und deren Auswertung.

Bevorzugterweise ist es möglich das System zur Steuerung und Erfassen von Messwerten eines mobilen Messgerätes ein durch die Datenerfassungseinrichtung an bestehenden Messgeräten nachrüstbar auszuführen.

Bevorzugterweise besitzt das System zur Steuerung und Erfassen von Messwerten eines mobilen Messgerätes zumindest ein System zum Erfassen der Ansteuersignale von Audioschallwandler zur Rückgewinnung der an den Menschen übertragenen Information.

Das System zur Steuerung und Erfassen von Messwerten eines mobilen Messgerätes ist vorteilhafterweise ausgebildet Daten in mobilen physiologien Messgeräten zu erfassen. Hierzu können insbesondere der Blutdruck, der Puls, die Temperatur, die Atmungsfrequenz, das EKG (d.h. die Stromspannungsschwankung im Körper bzw. im/am Herzen) und der Sauerstoffsättigungsgehalt des Blutes zählen.

Das System zur Steuerung und Erfassen von Messwerten eines mobilen Messgerätes ist vorteilhafterweise mobil ausgebildet und am Messobjekt angeordnet. Vorzugsweise kann diese auch in unmittelbarer räumlicher Nähe Messobjekt angeordnet sein, wobei unter "unmittelbarer räumlicher Nähe" vorzugsweise ein Bereich bis zu 10 m, besonders bevorzugterweise ca. 1 m. Unter "mobil" ist vorzugsweise ein Zustand zu verstehen, in welchem die Datenverarbeitungseinrichtung sowohl hinsichtlich ihrer räumlichen Abmessungen als auch hinsichtlich ihres Gewichts durch eine Person bzw. ein Lebewesen bewegbar ist. Vorzugsweise ist das mobile Messgerät leichter als ca. 1 kg. Die räumlichen Abmessungen bzw. die räumliche Ausdehnung der Datenerfassungseinrichtung ist vorteilhafterweise geringer als ca. 10 dm³ und besonders bevorzugterweise geringer als ca. 1 dm³.

Die Anordnung zur Steuerung von Messgeräten durch zentrale oder dezentrale Datenverarbeitungseinheiten ist vorteihafter so ausgelegt, dass die Steuerung ohne große Eingriffe in die Elektronik des Messgerätes erfolgt. Bevorzugterweise geschieht die Steuerung durch die Datenverarbeitungseinheiten indem die Bedienelemente (z.B. Schalter und Taster) durch Steuerelemente ersetzt oder parallelgeschaltet werden. Steuerelement sind zum Beispiel Relais, Bipolar- oder Feldeffekttransistoren.

Die Schnittstelle zu einer elektronischen Datenverarbeitungseinrichtung ist vorteilhafterweise drahtgebunden oder drahtlos (z.B. Funk) ausgeführt.

Das System zur Steuerung und Erfassen von Messwerten eines mobilen Messgerätes kann wahlweise vor der Weiterleitung der Daten an die zentrale oder dezentrale Datenverarbeitungseinrichtung eine Datenvorverarbeitung und/oder Speicherung der Daten vornehmen. Vorteilhafterweise ermöglicht dies z.B. eine Filterung der Daten und/oder ein Zwischenspeichern der Daten z.B. beim Fehlen der Verbindung zur Datenverarbeitungseinrichtung, bis eine bestimmte Anzahl von Daten gesammelt wurden oder die Daten durch die Datenverarbeitungseinrichtung abgerufen werden.

Die Erfindung wird nachfolgend anhand begleitender Zeichnungen bevorzugter Ausführungsformen beispielhaft beschrieben. Es zeigen:
- Fig. 1: typisches modernes Messgerät
- Fig. 2: Matrixanordnung eines Displays
- Fig. 3: LCD Display
- Fig. 4: System zum Rückgewinnen von dargestellten Informationen aus Displayansteuerungssignalen
- Fig. 5: Signalverläufe des Displays und der Signalwandlereinheit
- Fig. 6: Sechs sehr einfache Ausführungsformen für die benötigten Signalwandlereinheiten
- Fig. 7: eine Ausführungsform des Systems zur Steuerung und Erfassen von Messwerten eines mobilen Messgerätes

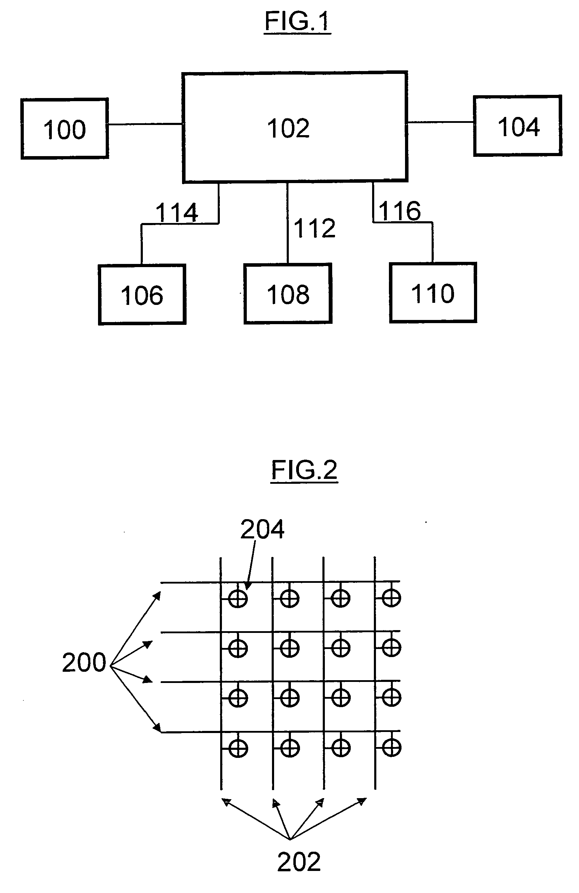

Fig. 1 ist typisches modernes Messgerät. Dieses kann beispielsweise ausgelegt sein, physiologische Parameter eines Lebewesens erfassen. Die zu messenden Daten werden durch einen Sensor bzw. ein Sensormittel (100) erfasst und an eine Datenverarbeitungseinheit (102) weitergeleitet. Die Bedienung erfolgt durch Bedienelemente (106) die mit der Datenverarbeitungseinheit (102) in Signalverbindung (114) stehen. Die Messwertausgabe erfolgt auf dem Display (108), das mit der Datenverarbeitungseinheit (102) in Signalverbindung (112) steht. Eine Audiosignalabgabe erfolgt durch den Audioschallwandler (110), der mit der Datenverarbeitungseinheit (102) in Signalverbindung (116) steht. Display (108) und Audioschallwandler (110) können alternativ oder auch kumulativ vorgesehen sein. Eine Energiequelle (104) z.B. eine Batterie dient zur Stromversorgung.

Fig. 2 zeigt die Matrixanordnung eines Displays (108). Das Display ist matrixartig aufgebaut und weist somit Zeilen (200) und Spalten (202) auf. Die Zeilen (200) sind vorzugsweise orthogonal zu den Spalten (202) angeordnet. Das Display weist eine Vielzahl von Anzeigeelementen (204) auf, um somit beispielsweise einen Bildpunkt darzustellen. Jedes Anzeigeelement (204) hat Signalverbindung mit einer Zeile und einer Spalte.

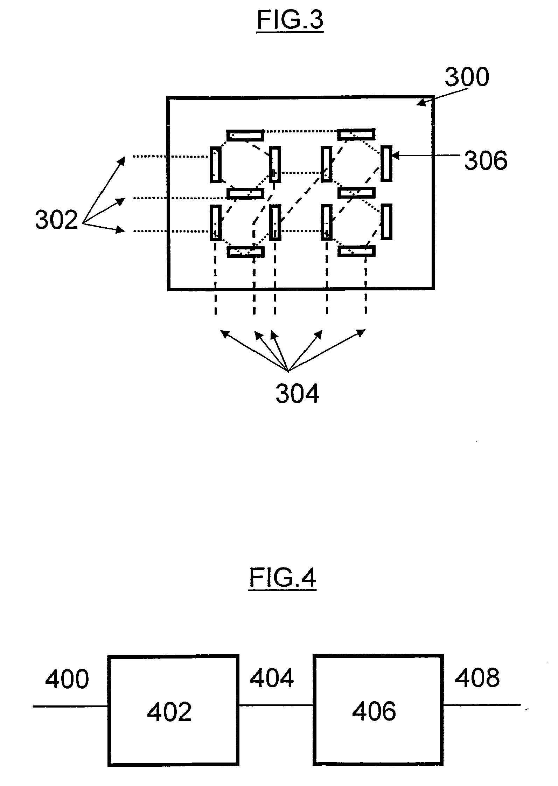

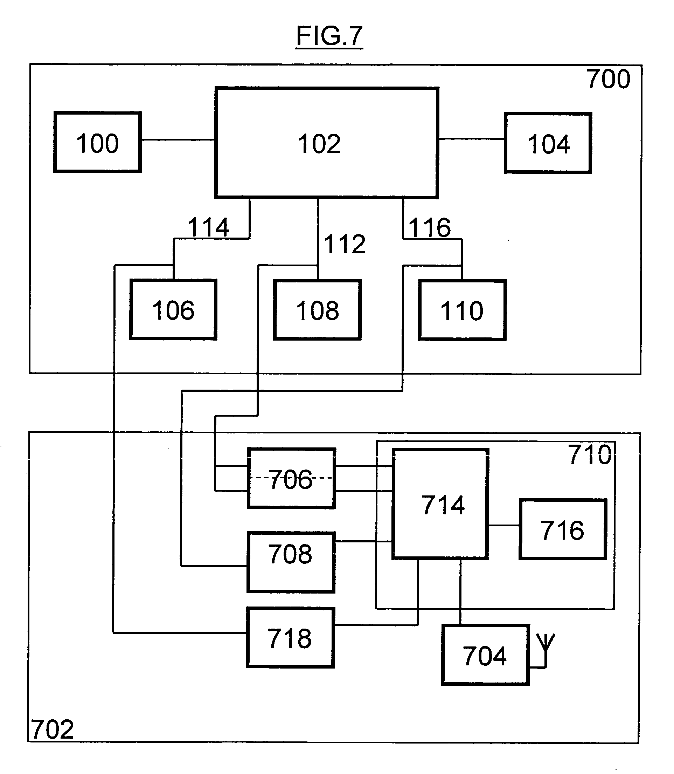

Fig. 3 zeigt ein LCD Display (Flüssigkristalldisplay) wie es später in den Erläuterungen zu Fig. 7 Verwendung findet. Das Flüssigkristalldisplay (300) hat auf der Oberseite eines Glasplattenträgers Zeilenleitungen (302), die kreuzungsfrei ausgeführt sind und die Displaysegmente (306) kontaktieren bzw. mit diesen in elektrischer Signalverbindung stehen. Auf der Unterseite des Glasplattenträgers verlaufen Spaltenleitungen (304), die ebenfalls kreuzungsfrei verlaufen und die Displaysegmente (306) kontaktieren bzw. mit diesen in elektrischer Signalverbindung stehen.

Fig. 4 zeigt eine bevorzugte Ausführungsform des erfindungsgemäßen Systems zum Rückgewinnen von dargestellten Informationen aus Displayansteuerungssignalen.

Die Displayansteuersignale zumindest einer Signalleitung eines Displays sind mit der Schnittstelle (400) verbunden bzw. werden von dieser abgegriffen und gelangen an die Signalwandlereinheit (402) bzw. werden zu dieser übertragen. Die Signalwandlereinheit (402) steht in Signalverbindung (404) mit der Auswerteeinheit (406). Diese besitzt eine Signalverbindung (408) zur drahtgebundenen oder drahtlosen Weiterleitung an zentrale oder dezentrale Datenverarbeitungseinrichtungen. Das System zum Erfassen der Ansteuersignale des Audioschallwandler zur Rückgewinnung der an den Menschen übertragenen Information nutzt die selbe Anordnung, nur dass das Schnittstelle (400) mit dem Ansteuersignal des Audioschallwandlers verbunden ist. Es versteht sich, daß das System ebenfalls beide Funktionen integrieren kann, so daß dargestellte Informationen aus Displayansteuerungssignalen und Ansteuersignale eines Audioschallwandlers rückgewonnen werden können.

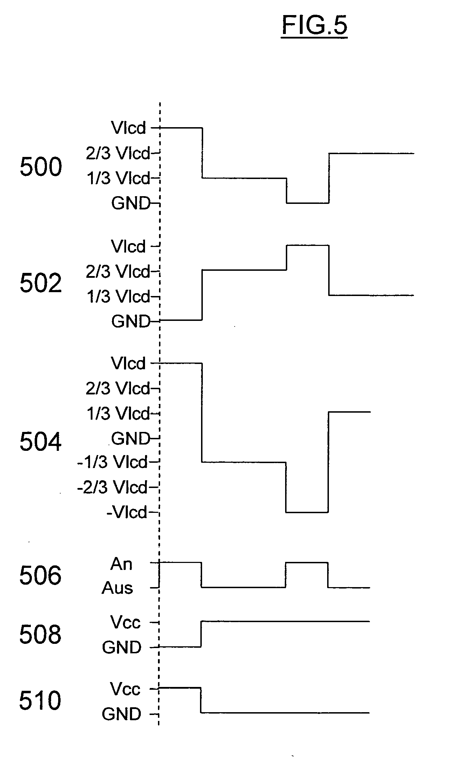

Fig. 5 zeigt die Signalverläufe des Displays und der Signalwandlereinheit. Dargestellt (500) ist eine Periode des Zeilenansteuersignals einer Zeile eines LCD Displays. Darunter zeigt (502) eine Periode eines Spaltenansteuersignals einer Spalte. Daraus ergibt sich durch Subtraktion der Signalverlauf am Anzeigeelement (504). Der Zustand des Anzeigelements ist in (506) dargestellt. Das angesteuerte Segment des Displays ist nur eingeschaltet wenn die entspechende Zeile Vlcd und die Spalte GND oder umgekehrt ist. (508) zeigt den Zeilenausgang der Signalwandlereinheit und (510) Spaltenausgang der Signalwandlereinheit unter Ausnutzung des gerade beschriebenen Effekts, dass nur die Maxima und Minima zur Auswertung benötigt werden.

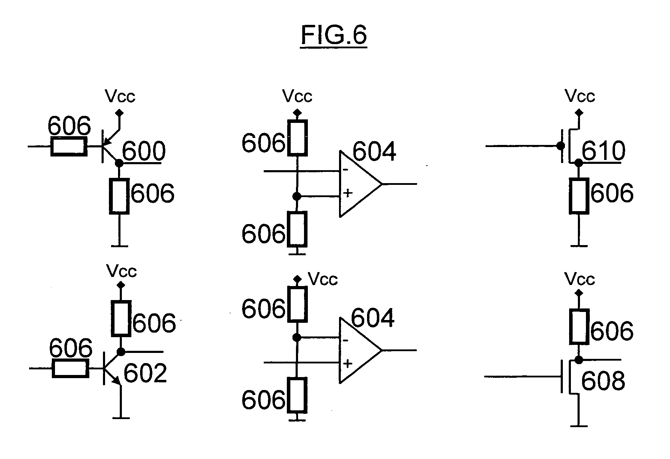

Fig. 6 zeigt sechs sehr einfache Ausführungsformen für die benötigten Signalwandlereinheiten. Alle Anordnungen werten das Eingangssignal nach Überbeziehungsweise Unterschreitung eines bestimmten Spannungspegels aus. Dazu kommen NPN (602) oder PNP (600) Bipolartransistoren mit Basis- und Kollektorwiderständen (606), N (608) oder P (610) Kanalfeldeffekttransistoren mit Drainwiderständen (606) oder Komparatoren (604) mit Widerstandsspannungsteilern (606) zum Einsatz.

Fig. 7 zeigt den Einsatz einer Ausführungsform des Systems zur Steuerung und Erfassen von Messwerten eines mobilen Messgerätes. Das ursprüngliche Messgerät (700) ist bereits in Fig.1 beschrieben. Das Systems zur Steuerung und Erfassen von Messwerten eines mobilen Messgerätes (702) steht in Signalverbindung mit dem ursprünglichen Messgerät (700). Die Signalwandlereinheit (706) des Displays greift auf die Signalverbindung zur Ansteuerung des Displays (112) zu. Innerhalb Signalwandlereinheit (706) ) des Displays werden die Zeilen und Spalten auf verschiedene Weise verarbeitet. Die Signalwandlereinheit (708) des Lautsprechers greift auf die Signalverbindung zur Ansteuerung des Lautsprechers (116) zu. Beide Signalwandlereinheiten sind mit der Auswerteeinheit (710) verbunden. Die Auswerteeinheit (710) besteht aus einer Prozessoreinheit (714) und einem Speicher (716). Der Speicher enthält den Programmcode und hält temporäre Daten während der Programmausführung. Weiterhin erfolgt in der Auswerteeinheit (710) eine Verarbeitung der Daten und gegebenenfalls eine Zwischenspeicherung der Messwerte. Weiterhin steht die Auswerteeinheit (710) in Verbindung mit der Steuerungs- und Abfrageanordnung (718). Diese erzeugt Steuersignale zur Steuerung des ursprünglichen Messgerätes (700), welche damit über die Signalverbindung der Bedienelemente (114) das ursprüngliche Messgerät (700) "ferngesteuert" und erfasst Signale der Signalverbindung der Bedienelemente (114) und gibt dadurch den Zustand der Bedienelement an die Auswerteeinheit (710) weiter. Zur Weitergabe der Messdaten und zum Empfang der Steuerungsinformation von/zu einer zentralen Datenverarbeitungseinrichtung steht die Prozessoreinheit (714) mit der drahtlosen Übertragungseinrichtung (704) in Verbindung.

Erfindungsgemäß ist also insbesondere ein System zum Erfassen der Ansteuersignale (116) von Audioschallwandler (110) zur Rückgewinnung der an den Menschen übertragenen Information vorgesehen, umfassend
- zumindest eine Schnittstelle (400), die auf mindestens eine Signalleitungen (116) des Audioschallwandlers (110) direkt zugreift,
- zumindest eine Signalwandlereinheit (402), welche mit dem Audioschallwandler (110) in Signalverbindung steht, um die Audioschallwandleransteuersignale (116) in digitale Signale umzusetzen,
- zumindest eine Auswerteeinheit (406), die mit der Signalwandeleinheit (402) in Signalverbindung steht und aus deren übertragener Information die Audioinformation und über mindestens eine Signalverbindung die zurückgewonnenen Messdaten Daten in ausgibt, wobei das System vorzugsweise
   als Nachrüstung an bestehende oder schon entwickelte Messgeräten ausgeführt ist, bevorzugterweise ausgebildet ist, um Daten in mobilen physiologischen Messgeräten zu erfassen und
   besonders bevorzugterweseise das Messgerät und die Auswertung mobil ausgebildet sind.

Ferner ist die Signalwandlereinheit vorzugsweise ausgelegt, den Zeit- und Spannungsverlauf der Displayansteuersignale in Digitale Signalverläufe umzusetzen und an die Auswerteeinheit weiterzuleiten, wobei die Signalwandlereinheit (402) vorzugsweise als Analog nach Digitalwandler der Signale ausgelegt ist,
besonders bevorzugterweise eine Klassifizierung der Signale in wenige Zustände vornehmen kann,
weiterhin vorzugsweise eine Klassifizierung der Signale in zwei Zustände vornehmen kann,
weiterhin vorzugsweise lediglich die Spannungsmaxima detektiert,
weiterhin vorzugsweise lediglich die Spannungsminima detektiert, und
weiterhin vorzugsweise die Spannungsminima und -maxima detektiert.

Ferner ist die Auswerteeinheit (406) vorteilhafterweise ausgelegt, die zeitliche Abfolge der Signale der Signalwandlereinheit zu erfassen und in digitale Informationen des Messwerts umzuwandeln, wobei die Auswerteeinheit (406) vorzugsweise als festverdrahtete Logik ausgebildet ist,
besonders bevorzugterweise als Mikroprozessorsystem mit Software ausgebildet,
weiterhin vorzugsweise die rückgewonnen Messdaten weiterleitet,
weiterhin vorzugsweise die rückgewonnenen Daten drahtgebunden überträgt,
weiterhin vorzugsweise rückgewonnenen Daten drahtlos überträgt, und
weiterhin vorzugsweise ganz oder teilweise in einen integrierten Schaltkreis integriert ist.

Vorteilhafterweise wird vor der Weiterleitung der Daten an die zentrale oder dezentrale Datenverarbeitungseinrichtung eine Datenvorverarbeitung und/oder Speicherung der Daten vorgenommen.

Weiterhin erfindungsgemäß vorgesehen ist ein System zur Steuerung und Erfassung von Messwerten eines mobilen Messgerätes, umfassend
- zumindest ein System zum Rückgewinnen von dargestellten Informationen aus Displayansteuerungssignalen und Weiterleitung an zentrale oder dezentrale Datenverarbeitungseinrichtung nach einem der vorhergehenden Ansprüche,
- zumindest eine Anordnung zur Steuerung von Messgeräte durch zentrale oder dezentrale Datenverarbeitungseinheiten,
- zumindest eine Schnittstelle zu einer elektronischen Datenverarbeitungseinrichtung, wobei das System
   vorteilhafterweise modular ausgebildet ist, um an bestehende oder vorbestimmte bzw. vorbestimmbare Messgeräte anordenbar zu sein,
   vorzugsweise ausgebildet ist, Daten in mobilen physiologischen Messgeräten zu erfassen,
   ferner vorzugsweise das System aus Messgerät und Auswertung mobil ausgebildet ist,
   ferner vorzugsweise rückgewonnene Daten drahtgebunden überträgt, und
   ferner vorzugsweise rückgewonnene Daten drahtlos überträgt.

Vorteilhafterweise ist die Auswerteeinheit ganz oder teilweise in einen integrierten Schaltkreis integriert ist.

Vorteilhafterweise wird vor der Weiterleitung der Daten an die zentrale oder dezentrale Datenverarbeitungseinrichtung eine Datenvorverarbeitung und/oder Speicherung der Daten vorgenommen.

Sämtliche weiteren Merkmale und Vorteile des erfindungsgemäßen Systems zum Rückgewinnen von dargestellten Informationen aus Displayansteuerungssignalen (112) und Weiterleitung an zentrale oder dezentrale Datenverarbeitungseinrichtung können ebenfalls in dem System zur Steuerung und Erfassung von Messwerten eines mobilen Messgerätes Anwendung finden.

### Bezugszeichenliste

- 100: Sensormittel
- 102: Datenverarbeitungseinheit
- 104: Energiequelle
- 106: Bedienelemente
- 108: Display
- 110: Audioschallwandler
- 112: Signalverbindung zur Ansteuerung des Displays
- 114: Signalverbindung der Bedienelemente
- 116: Signalverbindung des Audioschallwandlers

- 200: Zeilen
- 202: Spalten
- 204: Anzeigeelemente

- 300: Flüssigkristalldisplay
- 302: Zeilenleitungen
- 304: Spaltenleitungen
- 306: Displaysegment

- 400: Schnittstelle Signalverbindung
- 402: Signalwandlereinheit
- 404: Signalverbindung
- 406: Auswerteinheit
- 408: Signalverbindung

- 500: Zeilenansteuersignal
- 502: Spaltenansteuersignal
- 504: Signalverlauf am Anzeigeelement
- 506: Zustand des Anzeigelements
- 508: Zeilenausgang der Signalwandlereinheit
- 510: Spaltenausgang der Signalwandlereinheit

- 600: Bipolartransistor PNP
- 602: Bipolartransistor NPN
- 604: Komperator
- 606: Widerstand
- 608: N-Kanal Feldeffekttransistor
- 610: P-Kanal Feldeffekttransistor

- 700: Ursprüngliches Messgerät
- 702: System zur Steuerung und Erfassen von Messwerten eines mobilen Messgerätes
- 704: Drahtlose Übertragungseinheit
- 706: Signalwandlereinheit Display
- 708: Signalwandlereinheit Lautsprecher
- 710: Auswerteeinheit
- 714: Prozessoreinheit
- 716: Speicher
- 718: Steuerungs- und Abfrageanordnung

## Patentansprüche

1. System zum Rückgewinnen von dargestellten Informationen aus Displayansteuerungssignalen (112) und Weiterleitung an zentrale oder dezentrale Datenverarbeitungseinrichtungen, umfassend
- zumindest eine Schnittstelle (400), die ausgelegt ist, auf mindestens zwei Signalleitungen (112) eines Displays (108), welches ausgelegt ist Displaydaten darzustellen, mittelbar oder unmittelbar zuzugreifen,
- zumindest eine Signalwandlereinheit (402), welche mit der zumindest einen Schnittstelle (400) in Signalverbindung steht bzw. bringbar ist, um Ansteuersignale (112) des Displays (108) über die Schnittstelle (400) zu erfassen und in Wandlersignale umzusetzen,
- zumindest eine Auswerteeinheit (406), die mit der Signalwandlereinheit (402) in Signalverbindung (404) steht und Mittel aufweist, um aus den Wandlersignalen die Displaydaten zurückzugewinnen und über mindestens eine Signalverbindung (408) die zurückgewonnenen Displaydaten auszugeben.

2. System nach Anspruch 1, welches modular ausgebildet ist, um an standardisierte bzw. vorbestimmte oder vorbestimmbare Messgeräte anordenbar zu sein.

3. System nach mindestens einem der vorhergehenden Ansprüche, wobei das System mobil ausgebildet ist und vorzugsweise ausgebildet ist, Daten in mobilen physiologischen Messgeräten zu erfassen.

4. System nach mindestens einem der vorhergehenden Ansprüche, wobei die Signalwandlereinheit (402) ausgelegt ist, den Zeit- und Spannungsverlauf der Ansteuersignale (112) des Displays (108) in digitale Signalverläufe umzuwandeln und an die Auswerteeinheit (406) weiterzuleiten.

5. System nach Anspruch 4, wobei die Signalwandlereinheit (402) als Analog-nach-Digital-Wandler der Signale ausgelegt ist.

6. System nach einem der vorhergehenden Ansprüche, wobei die Signalwandlereinheit (402) ausgelegt ist, eine Klassifizierung der Wandlersignale in eine vorbestimmte oder vorbestimmbare Anzahl von vorzugsweise vorbestimmten oder vorbestimmbaren Zuständen vorzunehmen.

7. System nach Anspruch 6, wobei die Signalwandlereinheit (402) ausgelegt ist, eine Klassifizierung der Wandlersignale in zwei vorzugsweise vorbestimmte oder vorbestimmbare Zustände vorzunehmen.

8. System nach einem der vorhergehenden Ansprüche, wobei die Signalwandlereinheit (402) ausgelegt ist, von den Displayansteuerungssignalen (112), welche zur Darstellung von Information auf dem Display mittels eines matrixartigen Bildaufbaus ausgelegt sind, die Spannungsmaxima der den Zeilen zugeordneten Displayansteuerungssignalen (112) und die Spannungsminima der den Spalten zugeordneten Displayansteuerungssignalen (112) zu detektieren.

9. System nach einem der vorhergehenden Ansprüche, wobei die Signalwandlereinheit (402) ausgelegt ist, von den Displayansteuerungssignalen (112), welche zur Darstellung von Information auf dem Display mittels eines matrixartigen Bildaufbaus ausgelegt sind, die Spannungsmaxima der den Spalten zugeordneten Displayansteuerungssignalen (112) und die Spannungsminima der den Zeilen zugeordneten Displayansteuerungssignalen (112) zu detektieren.

10. System nach einem der vorhergehenden Ansprüche, wobei die Signalwandlereinheit (402) ausgelegt ist, von den Displayansteuerungssignalen (112), welche zur Darstellung von Information auf dem Display mittels eines matrixartigen Bildaufbaus ausgelegt sind, alle Spannungsminima und -maxima der den Spalten und Zeilen zugeordneten Displayansteuerungssignalen (112) zu detektieren.

11. System nach einem der vorhergehenden Ansprüche, wobei die Signalwandlereinheit (402) Zeilen- und/oder Spaltenwandler aufweist, deren Anzahl vorzugsweise geringer als die Anzahl der Zeilen bzw. Spalten ist, welche den Displayansteuerungssignalen (112) zur Darstellung von Information auf dem Display mittels eines matrixartigen Bildaufbaus entsprechen.

12. System nach einem der vorhergehenden Ansprüche, wobei die Signalwandlereinheit (402) einen einzigen Zeilen- und/oder Spaltenwandler aufweist.

13. System nach einem der Ansprüche 11 oder 12, wobei die Signalwandlereinheit (402) Mittel zur adaptiven Rückgewinnung der nicht gewandelten Spaltensignale aufweist.

14. System nach einem der vorhergehenden Ansprüche, wobei die Auswerteeinheit (406) ausgelegt ist, die zeitliche Abfolge der Signale der Signalwandlereinheit (402) zu erfassen und in die Displaydaten, vorzugsweise digitale Informationen eines Messwerts, umzuwandeln.

15. System nach einem der vorhergehenden Ansprüche, wobei die Auswerteeinheit (406) ausgelegt ist, im Zeitmultiplex eintreffende Daten zu erfassen und zumindest temporär zu speichern, bis ein komplettes Abbild der Displaydaten vorliegt ,und diese dann elektronisch zu analysieren.

16. System nach einem der vorhergehenden Ansprüche, wobei die Auswerteeinheit (406) als festverdrahtete Logik und/oder als Mikroprozessorsystem mit Software ausgebildet ist.

17. System nach einem der vorhergehenden Ansprüche, wobei die Auswerteeinheit (406) Übertragungsmittel aufweist, um die rückgewonnen Displaydaten bzw. Messdaten weiterzuleiten.

18. System nach einem der vorhergehenden Ansprüche, wobei die Auswerteeinheit (406) Komparatormittel aufweist, um eine Veränderung des Displayinhalts zu erfassen und diese weiterzuleiten, wobei die Komparatormittel ausgelegt sind, anhand der Veränderung des Displayinhaltes Zustandsänderungen der Displaydaten zu erkennen .

19. System nach einem der vorhergehenden Ansprüche, wobei die Auswerteeinheit (406) die rückgewonnenen Displaydaten bzw. Daten drahtgebunden und/oder drahtlos mittels Übertragungsmittel überträgt.

20. System nach einem der vorhergehenden Ansprüche, wobei die Signalwandlereinheit (402) und/oder die Auswerteeinheit (406) ganz oder teilweise in einen integrierten Schaltkreis integriert ist.

21. System nach mindestens einem Ansprüche 1 bis 20, wobei vor der Weiterleitung der Daten an die zentrale oder dezentrale Datenverarbeitungseinrichtung eine Datenvorverarbeitung und/oder Speicherung der Daten vorgenommen wird.

22. System zur Steuerung und Erfassen von Messwerten eines mobilen Messgerätes, umfassend
- zumindest ein System zum Rückgewinnen von dargestellten Informationen aus Displayansteuerungssignalen und Weiterleitung an zentrale oder dezentrale Datenverarbeitungseinrichtung nach einem der vorhergehenden Ansprüche,
- zumindest eine Anordnung zur Steuerung von Messgeräte durch zentrale oder dezentrale Datenverarbeitungseinheiten,
- zumindest eine Schnittstelle zu einer elektronischen Datenverarbeitungseinrichtung
